# EUROPEAN PATENT SPECIFICATION

(11) **EP 1 669 059 B1**
(45) Date of publication and mention of the grant of the patent: **21.05.2008**
(21) Application number: 05257652.7
(22) Date of filing: 12.12.2005
(51) Int. Cl.: A61K 8/22, A61K 8/19, A61Q 11/02

(54) **Photocatalytic bleaching agent for teeth based on bismuth vanadate and bleaching method**
Photokatalytisches Bleichmittel für Zähne auf der Basis von Bismuthvanadat und Bleichverfahren
Agent photocatalytique de blanchiment pour les dents à base de vanadate de bismuth et procédé de blanchissement

(30) Priority: 13.12.2004 JP 2004359616
(43) Date of publication of application: 14.06.2006
(73) Proprietor: MITSUBISHI GAS CHEMICAL COMPANY, INC., Chiyoda-ku, Tokyo 100-8324 (JP)
(72) Inventor: Kakuda, Minoru, c/o Mitsubishi Gas Chem.Co., Inc., Katsushika-ku Tokyo (JP); Kurata, Hiroshi, c/o Mitsubishi Gas Chem.Co., Inc., Katsushika-ku Tokyo (JP)
(74) Representative: Gillard, Richard Edward

(56) References cited:
- EP-A- 1 048 291
- EP-A- 1 192 933
- US-B1- 6 403 107

## Description

### TECHNICAL FIELD

The present invention relates to a material and a method for bleaching and removing colored sediment on (stained or discolored) teeth. More specifically, the present invention relates to a method for bleaching discolored teeth comprising the steps of applying a bleaching material of a specific composition having photocatalytic action on the surface of discolored teeth, and bleaching the teeth based on the photocatalytic action that is produced by irradiating that surface area with light; and a bleaching material for use in the above-described method.

### BACKGROUND ART

There has been increasing demand in recent years for improvements in the contour, alignment, and integrity of teeth in dental therapy, which may be referred to as cosmetic improvements. Recently, there have been more cases of patients seeking dental therapy based on desires for whiter teeth, which is increasingly regarded by young women as an important element of beauty. The causes of dental discoloration and pigmentation or staining are generally classified into the so-called extrinsic factors, such as sedimentation of colored substances (tobacco, tea, etc.), pigment generating bacteria, the discoloration of repair materials (mainly, composite resins), metal salts (mainly, amalgams, silver nitrate and ammonia silver), and intrinsic factors, such as aging, chemicals or drugs (such as fluorine and tetracycline), dysmetabolism and hereditary diseases, and dental injuries.

Several methods for cosmetic improvement of discolored teeth have been proposed to date, among which bleaching can be said a highly effective method for the preservation of dentin, though this method has drawbacks such as occasional recurrence. Bleaching is essentially a method of decolorizing colored substances by means of a chemical reaction. Bleaching materials comprising a variety of chemical agents and methods of bleaching using such materials have been reported for vital teeth bleaching and non-vital teeth bleaching.

For bleaching materials and methods to be applied to teeth, the following conditions are required:
(a) remarkable bleaching effect,
(b) no toxicity in the chemical agents used,
(c) simple to handle/operate,
(d) no deterioration in the physical property of dentine after treatment,
(e) effective for both vital and non-vital teeth, and
(f) rapid bleaching effect.
However, in conventional bleaching materials and methods, 30-35 wt % aqueous hydrogen peroxide which is highly tissue-corrosive has been used as the primary chemical agent, and bleaching has been performed based on the oxidation action of this agent. Therefore, these conventional materials and methods have drawbacks in terms of simple handling/operation and safety.

Under the circumstances around bleaching methods as described so far, novel bleaching materials and bleaching methods are demanded which are excellent in safety and simple handling/operation, and yet produce remarkable effect on both vital and non-vital teeth in a short period of time.

As such a bleaching material/bleaching method, a bleaching material comprising a combination of titanium dioxide having photocatalytic action and an aqueous hydrogen peroxide of low concentration has been reported effective (see Japanese Patent No. 3030380 (corresponding US Patent No. 6231343)).

On the other hand, with respect to photocatalysts, visible light-responsive photocatalysts have been studied and developed aggressively in order to decompose water or to make harmful substances harmless or clean, utilizing sunlight comprising abundant visible light. A great number of photocatalysts have been proposed which include various composite oxides (Japanese Unexamined Patent Publications Nos. 2001-002419; 2003-33661; 2003-251197; and 2004-24936). Among all, BiVO₄ photocatalyst disclosed in Japanese Unexamined Patent Publications Nos. 2001-002419 and 2004-24936 is known to be effective in decomposing organic environmental pollutants in aqueous systems.

US Patent No. 6,403,701 discloses a coloured composition for topical application which contains bismuth vanadate in a hydrophilic continuous phase. The bismuth vanadate is present as a colouring agent and the composition relies upon the intense, saturated yellow colour and stability of the bismuth vanadate.

### DISCLOSURE OF INVENTION

It is an object of the present invention to provide a novel bleaching material and a novel bleaching method both of which are applicable to cosmetic improvement of discolored teeth. It is another object of the present invention to provide a bleaching material which is still easier to use by combining BiVO₄ having photocatalytic action with a compound which generates hydrogen peroxide in an aqueous solution to thereby reduce the time required for bleaching treatment.

The present inventors have paid attention to the decomposition action of the above-mentioned BiVO₄ photocatalyst on organic environmental pollutants, and considered that BiVO₄ photocatalyst should also act effectively on colored organic sediments on the surface of teeth.

As a result of intensive and extensive investigations into the above-described bleaching material, the inventors have found that the time required for bleaching treatment can be reduced by using BiVO₄ having photocatalytic action. Thus, the present invention has been achieved. The present invention relates to a bleaching material for bleaching teeth by applying a solution or paste comprising BiVO₄ photocatalyst and a compound which generates hydrogen peroxide in an aqueous solution onto the surface of discolored teeth, and irradiating the surface with light, thereby bleaching the teeth by resultant photocatalytic action, wherein the material comprises as active ingredients a combination of BiVO₄ photocatalyst and a compound which generates hydrogen peroxide in an aqueous solution, as well as a method of bleaching using the above bleaching material.

### BEST MODE FOR CARRYING OUT THE INVENTION

More specifically, the present invention provides the following bleaching materials and bleaching method for discolored teeth.
(1) A bleaching material for discolored teeth, comprising as active ingredients BiVO₄ photocatalyst and a compound which generates hydrogen peroxide in an aqueous solution.
(2) A bleaching material for discolored teeth by applying a solution or paste comprising BiVO₄ photocatalyst and a compound which generates hydrogen peroxide in an aqueous solution onto the surface of discolored teeth, and irradiating the surface with light, thereby bleaching the teeth by resultant photocatalytic action, wherein the material comprises as active ingredients a combination of BiVO₄ photocatalyst and a compound which generates hydrogen peroxide in an aqueous solution.
(3) The bleaching material according to (1) or (2) above, wherein the material comprises 0.01-20% by weight of BiVO₄ photocatalyst relative to the total weight of the material.
(4) The bleaching material according to (1) or (2) above, wherein the compound which generates hydrogen peroxide in an aqueous solution comprises hydrogen peroxide.
(5) The bleaching material according to (1) or (2) above, wherein the material comprises 35% by weight of the compound which generates hydrogen peroxide in an aqueous solution relative to the total weight of the material at the maximum.
(6) The bleaching material according to (1) or (2) above, wherein the material comprises 1-10% by weight of the compound which generates hydrogen peroxide in an aqueous solution relative to the total weight of the material.
(7) The bleaching material according to (1) or (2) above, wherein the material further comprises a thickening agent.
(8) The bleaching material according to (7) above, wherein the thickening agent is a layer-structured clay mineral.
(9) A method of bleaching teeth, comprising applying a solution or paste comprising BiVO₄ photocatalyst and a compound which generates hydrogen peroxide in an aqueous solution onto the surface of discolored teeth, and irradiating the surface with light, thereby bleaching the teeth by resultant photocatalytic action.
(10) The method according to (9) above, wherein visible light is irradiated as the light.

According to a preferred embodiment of the present invention, a desired effect can be obtained in a shorter period of time than in conventional methods by using the teeth bleaching material of the invention comprising BiVO₄ having photocatalytic action and a compound which generates hydrogen peroxide in an aqueous solution. Thus, the present invention can provide a simple to use, novel bleaching material and a bleaching method using the material.

Hereinbelow, the present invention will be described in more detail.

The bleaching material of the invention for discolored teeth is characterized by comprising BiVO₄ photocatalyst and a compound which generates hydrogen peroxide in an aqueous solution. In the present invention, the term discoloration refers to sedimentation of colored matter on teeth and is used in a broad range of meaning including staining.

BiVO₄ used in the present invention is a composite oxide having photocatalytic function. BiVO₄ used in the present invention is a known material and may be synthesized by a conventional solid phase reaction method, i.e., by mixing oxides of individual metal components (raw materials) in a ratio to give a composition of interest and burning the mixture in air under normal atmospheric pressure. Alternatively, BiVO₄ may be prepared by a homogeneous precipitation method or the like in which NH₄VO₃ is reacted with Bi(NO₃)₃ in the presence of ammonia, urea or the like. Further, BiVO₄ may be prepared according to methods disclosed in, for example, Japanese Unexamined Patent Publications Nos. 2001-002419 and 2004-24936.

The shape of BiVO₄ used in the present invention is preferably fine particles with a large surface area from the viewpoint of utilizing light effectively. When particles of the prepared BiVO₄ are large and thus the surface area is small, the particles may be ground in a ball mill or the like to make the particle size smaller. Generally, the particle size is 1 nm to 500 µm, preferably 1 nm to 50 µm.

Further, the BiVO₄ photocatalyst used in the present invention may be modified in a manner as conventionally used in the preparation of photocatalysts. For example, the BiVO₄ photocatalyst may be allowed to carry as a promoter a noble metal such as Pt, a transition metal such as Ni, an oxide such as NiOₓ (wherein x is a value exceeding 0 and not exceeding 1), IrO₂ or RuO₂. Such modification may be achieved by an impregnation method or photo-electrodeposition method. In an impregnation method, for example, an aqueous solution of a chloride, nitrate salt or the like of a metal of interest is used to impregnate the target composite oxide with the metal. The resultant composite oxide is dried at 100-200°C for about 2 hours, and then burnt at 800°C or below, preferably at 200-500°C under a reducing atmosphere and/or an oxidizing atmosphere for 2 to 5 hours. The amount of the promoter is preferably 0.01-10% by weight, more preferably 0.1-5% by weight relative to the total weight of BiVO₄ photocatalyst.

The amount of BiVO₄ photocatalyst in the bleaching material of the present invention is preferably 0.001-20% by weight, more preferably 0.01-5% by weight relative to the total weight of the bleaching material. When the amount of BiVO₄ photocatalyst is too small, it may require a long time to achieve favorable results depending on the degree of discoloration of the teeth. On the other hand, when the amount of BiVO₄ photocatalyst is too large, bleaching effect may be lowered because photopermeability becomes worse.

As the compound which generates hydrogen peroxide in an aqueous solution used in the present invention, any compound which would generate hydrogen peroxide when dissolved in water may be used. Specific examples of this compound include, but are not limited to, hydrogen peroxide, perborates, percarbonates, perphosphorates, persulfates, calcium peroxide, magnesium peroxide and urea peroxide. Among all, hydrogen peroxide is preferably used in the present invention. A mixture of two or more of these compounds that generate hydrogen peroxide may also be use.

The teeth bleaching material of the present invention is capable of producing a remarkable bleaching effect even when the concentration of a compound which generates hydrogen peroxide in an aqueous solution is sufficiently decreased compared to the concentration used in conventional bleaching materials. That is, the content of a compound which generates hydrogen peroxide in an aqueous solution is 35% by weight at the maximum, preferably 1-30% by weight, more preferably 1-10% by weight, still more preferably 1-5% by weight relative to the total weight of the bleaching material. Even when this compound is combined in the bleaching material exceeding the upper limit of this range, no remarkable difference is seen in bleaching effect. Further, such a high content is not advantageous from the viewpoint of safety.

Usually, the bleaching material of the present invention is prepared by mixing BiVO₄ photocatalyst into an aqueous solution of a compound which generates hydrogen peroxide in an aqueous solution (e.g., aqueous hydrogen peroxide) and used in the form of solution or paste. However, the form of the bleaching material of the present invention is not limited to these forms as long as the material is prepared by mixing the above-described components.

When the bleaching material of the present invention is used as a paste-like bleaching material, it is preferable to use a thickening agent. Either inorganic or organic thickening agents may be used. However, when an organic thickening agent is used, one hard to decompose by the photocatalytic action is preferably used.

As inorganic thickening agents, inorganic clay minerals are preferable. More preferably, layer-structured clay minerals are used. Generally, inorganic clay minerals are roughly classified into fibrous structure type, non-crystal structure type, mixed layer structure type and the above-mentioned layer structure type.

Layer-structured clay minerals take water molecules into the unit space between layers and then become swollen. By utilizing this property, the hydrogen peroxide present in the bleaching material is retained in contact with the surface of the discolored teeth. In the present invention, it is preferable to use a layer-structured clay mineral having such a property of swelling in the presence of water.

Even if a clay mineral is of fibrous structure type or non-crystal structure type, it is possible to realize a swollen state by agitating it in a high speed agitator after addition of water thereto. A layer-structured clay mineral is advantageous because it does not require such special equipment.

Specific examples of inorganic clay minerals include, but are not limited to, dickite, nacrite, kaolinite, anorthite, halloysite, metahalloysite, chrysotile, lizardite, serpentine, antigorite, beidellite, montmorillonite, sauconite, stevensite, nontronite, saponite, hectorite, vermiculite, smecnite, sepiolite, nacrite, illite, sericite, glauconite-montmorillonite, roselite-montmorillonite, chlorite-vermiculite, illite-montmorillonite, halloysite-montmorillonite and kaolinite-montmorillonite.

Among the above inorganic clay minerals, particularly preferable layer structured inorganic clay minerals are montmorillonite, sauconite, smecnite, stevensite, beidellite, nontronite, saponite, hectorite, vermiculite and nacrite.

These inorganic clay minerals may be either natural products or synthesized products. Examples of synthesized products include synthetic sodium lithium magnesium silicate (Laponite). A mixture of two or more of these clay minerals may also be used.

On the other hand, as organic thickening agent, water-soluble polymers are preferable. More preferably, thickening agents used as food additives are used from the viewpoint of safety. Especially preferable thickening agents used as food additives include, but are not limited to, sodium alginate, propyleneglycol alginate, carboxymethylcellulose sodium, sodium starch glycolate, sodium starch phosphate ester, methylcellulose and sodium polyacrylate. Among all, sodium polyacrylate, methylcellulose and carboxymethylcellulose sodium having excellent preservation stability are more preferable. These thickening agents may be either natural products or synthetic products. A mixture of two or more of these thickening agents may also be use.

The amount of the thickening agent is preferably 0.01-10% by weight, more preferably 0.1-5% by weight relative to the total weight of the bleaching material.

The amount of thickening agent is selected to give a viscosity of 0.001-100 Pa·s, preferably 0.001-50 Pa·s, more preferably 0.001-10 Pa·s, still more preferably 0.002-0.10 Pa·S. The viscosity of the bleaching material may be measured with a single cylinder-type rotational viscometer. The viscosity within the above-mentioned range does not cause any dropping of the bleaching material applied to the teeth surface even forming an angle of 45 degrees against the horizontal plane. The thickening agent used in the invention may be either a natural product or a synthetic product. A mixture of two or more thickening agents may also be used. The amount of such a thickening agent added to the bleaching material of the invention is preferably 0.01-10% by weight, more preferably 0.1-5% by weight.

When the bleaching material of the present invention is applied onto the surface of discolored teeth, it is preferable to directly apply the bleaching material of the invention onto the surface of discolored teeth. At this time, cloth, paper, glass cloth, ceramic paper, organic gel, inorganic gel, or the like may be impregnated with the bleaching material of the invention and then applied to the teeth. BiVO₄ photocatalyst and a compound which generates hydrogen peroxide in an aqueous solution may be prepared separately and mixed appropriately before application onto the surface of discolored teeth.

The bleaching material of the invention is applied directly onto the surface of teeth and then light is irradiated once or several times. At this time, it is also possible to apply cloth or the like impregnated with the bleaching material of the invention onto the surface of teeth and irradiate light in that state. With respect to the light to be irradiated, preferably the light has wavelengths that are absorbed by the composite oxide in the bleaching material and can induce photocatalytic action in the composite oxide. And yet it is preferable for the light to have wavelengths with less side effect on the living body. As a specific example of such light, light comprising wavelengths of 300 nm or more, more preferably light in a visible range comprising wavelengths of 380 nm or more is used. Examples of the light source (radiation instrument) used in the present invention generally include, but are not limited to, white heating lamps, fluorescent light lamps, halogen lamps, black light, metal halide lamps, xenon lamps, mercury lamps, UV lamps, LED (light-emitting diodes) and semiconductor lasers. Light emitted from these sources may be passed through an appropriate filter to cut unnecessary wavelengths and give specific wavelengths.

The number of times of application of the bleaching material and right irradiation may be adjusted appropriately depending on the degree of discoloration.

According to a preferred embodiment of the invention, the bleaching material and bleaching method of the invention are effective for both non-vital and vital teeth and capable of producing remarkable effect in bleaching those teeth safely and simply.

The present patent application claims priority based on Japanese Patent Application No. 2004-359616 filed on December 13, 2004. The contents of the specification of this Japanese Patent Application are incorporated in the present specification by reference. Also, documents and patent publications, patents and other patent documents cited in the present specification are incorporated by the present specification by reference.

### EXAMPLES

Hereinbelow, the present invention will be described in more detail with reference to the following Examples. However, the present invention is not limited by the following Examples..

The BiVO₄ photocatalysts (samples A to C) were prepared as described below.

### Sample A

Bi₂O₃ (Kanto Chemical; special grade) (7.19 g) and V₂O₅ (Kanto Chemical; special grade) (2.81 g) were mixed. The mixture was placed in an aluminium pot and burned in a muffle furnace at 830°C for 12 hours under air flow. The burned product was cooled to thereby obtain 9.75 g of yellow sample.

### Sample B

Using 2 mol/L of nitric acid solution, 0.12 mol/L of NH₄VO₃ (Kanto Chemical; special grade) solution and 0.12 mol/L of Bi(NO₃)₃·5H₂O (Wako Purechemical; special grade) solution were prepared separately. Subsequently, 200 ml each of the 0.12 mol/L of NH₄VO₃ solution and 0.12 mol/L of Bi(NO₃)₃·5H₂O solution were placed in a 500 ml eggplant-shaped flask to prepare a homogeneous solution. To this solution, 14.2 g of urea (Kanto Chemical; special grade) was added, dissolved and retained at 90°C for 3 hours. Gradually, reddish brown precipitate was confirmed. Then, the solution was cooled to 50°C or below, and the precipitate was recovered by vacuum filtration. The precipitate was washed with pure water and then dried to thereby obtain 4.0 g of reddish brown crystals.

### Sample C

A commercial BiVO₄ (purity 99.9%; Soekawa Chemical) was used.

As model tests to examine bleaching effect, the following (1) methylene blue bleaching test and (2) discolored teeth bleaching test were carried out.

### (1) Methylene Blue Bleaching Test

Methylene blue (blue dye) was dissolved in a bleaching material at 10 ppm to prepare a test solution, which was then placed in a quartz cell of 1 cm square. Light was irradiated using a dental, visible light irradiating unit (Optilux™ 501; Demetron) (radiation wavelength: 360-530 nm). The time required to reach 90% decomposition of methylene blue was observed. The results are summarized in Table 1.

### (2) Discolored Teeth Bleaching Test

Bleaching test for discolored teeth (pulled out teeth) was performed by the procedures described below using a bleaching material. 1) Tartar, calculus, tar and the like were removed with an ultrasonic scaler for preparation. 2) The surfaces of teeth were cleaned by a common method using a rubber cap or the like, and were dried. 3) The teeth were then temporarily moisture-proofed. 4) A bleaching material is applied onto the surfaces of teeth, and then the surfaces of teeth were irradiated with a dental, visible light irradiating unit (Optilux^{™} 501; Demetron) (radiation wavelength: 360-530 nm). 5) Light was irradiated for 2 minutes/time. Every time fresh bleaching material was applied and then light was irradiated. These operations were repeated 5 times (total irradiation time: 10 minutes). The surfaces of teeth before and after bleaching were measured with a spectroscopic color difference meter (Model SE-2000; Nippon Denshoku) and changes in discolored teeth were expressed with L* (lightness), a* (reddish tinge) and b* (yellowish tinge). The results are summarized in Table 2.

### [Example 1]

A bleaching material comprising 0.6% by weight of sample A and 3.5% by weight of hydrogen peroxide was prepared using water as a solvent. With the resultant bleaching material, methylene blue bleaching test and discolored teeth bleaching test were carried out (Tables 1 and 2).

### [Example 2]

A bleaching material comprising 0.6% by weight of sample B and 3.5% by weight of hydrogen peroxide was prepared using water as a solvent. With the resultant bleaching material, methylene blue bleaching test and discolored teeth bleaching test were carried out (Tables 1 and 2).

### [Example 3]

A bleaching material comprising 0.6% by weight of sample C and 3.5% by weight of hydrogen peroxide was prepared using water as a solvent. With the resultant bleaching material, methylene blue bleaching test and discolored teeth bleaching test were carried out (Table 1).

### [Comparative Example 1]

A bleaching material was prepared in the same manner as in Example 1 except that a commercial titanium dioxide (product name: MT-150A; Teika Co.) was used as a composite oxide having photocatalytic reaction. With the resultant bleaching material, methylene blue bleaching test and discolored teeth bleaching test were carried out (Tables 1 and 2).

**Table 1. Results of Methylene Blue Bleaching Test**

| | Photocatalyst | Time required for 90% decomposition (min) |
|---|---|---|
| Example 1 | Sample A | 1.0 |
| Example 2 | Sample B | 1.5 |
| Example 3 | Sample C | 1.5 |
| Comparative Example 1 | MT-150A | 3.0 |

**Table 2. Results of Discolored Teeth Bleaching Test**

| | Photocatalyst | Color difference | | | | | |
|---|---|---|---|---|---|---|---|
| | | Before bleaching | | | After 10 min radiation | | |
| | | L* | a* | b* | L* | a* | b* |
| Example 1 | Sample A | 62 | -2 | 2 | 65 | -2 | -1 |
| Example 2 | Sample B | 58 | -2 | -2 | 62 | -2 | -3 |
| Comparative Example 1 | MT-150A | 64 | -2 | 2 | 66 | -2 | 0 |

| | | | | | | | |
|---|---|---|---|---|---|---|---|
| L*: lightness +L* (light) - -L* (dark) a*: reddish tinge +a* (red) - -a* (green) b*: yellowish tinge +b* (yellow) - -b* (blue) | | | | | | | |

### INDUSTRIAL APPLICABILITY

The bleaching material and bleaching method of the present invention are capable of bleaching discolored teeth safely and simply. Thus, they are very useful in cosmetic dental treatment.

## Claims

1. A bleaching material for discolored teeth, comprising as active ingredients BiVO₄ photocatalyst and a compound which generates hydrogen peroxide in an aqueous solution.

2. The bleaching material according to claim 1, wherein the material comprises 0.01-20% by weight of BiVO₄ photocatalyst relative to the total weight of the material.

3. The bleaching material according to claim 1 or 2, wherein the compound which generates hydrogen peroxide in an aqueous solution comprises hydrogen peroxide.

4. The bleaching material according to any preceding claim wherein the material comprises up to and including 35% by weight of the compound which generates hydrogen peroxide in an aqueous solution relative to the total weight of the material at the maximum.

5. The bleaching material according to claim 4, wherein the material comprises 1-10% by weight of the compound which generates hydrogen peroxide in an aqueous solution relative to the total weight of the material.

6. The bleaching material according to any preceding claim wherein the material further comprises a thickening agent.

7. The bleaching material according to claim 6, wherein the thickening agent is a layer-structured clay mineral.

8. A method of bleaching teeth, comprising applying a solution or paste comprising the bleaching material according to any preceding claim onto the surface of discolored teeth, and irradiating said surface with light, thereby bleaching the teeth by resultant photocatalytic action.

9. The method according to claim 8, wherein visible light is irradiated as the light.

## Patentansprüche

1. Bleichendes Material für verfärbte Zähne, umfassend als Wirkbestandteile BiVO₄-Photokatalysator und eine Verbindung, die in einer wässrigen Lösung Wasserstoffperoxid erzeugt.

2. Bleichendes Material nach Anspruch 1, wobei das Material 0,01 - 20 Gew.-% BiVO₄-Photokatalysator, bezogen auf das Gesamtgewicht des Materials, umfasst.

3. Bleichendes Material nach Anspruch 1 oder 2, wobei die Verbindung, die in einer wässrigen Lösung Wasserstoffperoxid erzeugt, Wasserstoffperoxid umfasst.

4. Bleichendes Material nach einem vorangehenden Anspruch, wobei das Material bis zu und einschließlich 35 Gew.-% der Verbindung, die in einer wässrigen Lösung Wasserstoffperoxid erzeugt, bezogen auf das Gesamtgewicht des Materials höchstens bzw. bei dem Maximum, umfasst.

5. Bleichendes Material nach Anspruch 4, wobei das Material 1 - 10 Gew.-% der Verbindung, die in einer wässrigen Lösung Wasserstoffperoxid erzeugt, bezogen auf das Gesamtgewicht des Materials, umfasst.

6. Bleichendes Material nach einem vorangehenden Anspruch, wobei das Material weiterhin ein Verdickungsmittel umfasst.

7. Bleichendes Material nach Anspruch 6, wobei das Verdickungsmittel ein Schicht-strukturiertes Ton-Mineral ist.

8. Verfahren zum Bleichen von Zähnen, umfassend Auftragen einer Lösung oder Paste, umfassend das bleichende Material nach einem vorangehenden Anspruch, auf die Oberfläche von verfärbten Zähnen und Bestrahlen der Oberfläche mit Licht, wodurch die Zähne durch die sich ergebene photokatalytische Wirkung gebleicht werden.

9. Verfahren nach Anspruch 8, wobei mit sichtbarem Licht als das Licht bestrahlt wird.

## Revendications

1. Agent de blanchiment conçu pour blanchir des dents jaunies, comprenant, comme ingrédients actifs, un photocatalyseur à base de vanadate de bismuth BiVO₄ et un composé qui, dans une solution aqueuse, donne du peroxyde d'hydrogène.

2. Agent de blanchiment, conforme à la revendication 1, lequel agent contient du photocatalyseur à base de BiVO₄ en une quantité représentant de 0,01 à 20 % de son poids total.

3. Agent de blanchiment, conforme à la revendication 1 ou 2, dans lequel le composé qui donne du peroxyde d'hydrogène dans une solution aqueuse comprend du peroxyde d'hydrogène.

4. Agent de blanchiment, conforme à l'une des revendications précédentes, lequel agent contient du composé donnant du peroxyde d'hydrogène dans une solution aqueuse en une quantité représentant jusqu'à 35 %, limite comprise, de son poids total.

5. Agent de blanchiment, conforme à la revendication 4, lequel agent contient d u composé donnant du peroxyde d'hydrogène dans une solution aqueuse en une quantité représentant 1 à 10 % de son poids total.

6. Agent de blanchiment, conforme à l'une des revendications précédentes, lequel agent contient en outre un agent épaississant.

7. Agent de blanchiment, conforme à la revendication 6, dans lequel l'agent épaississant est un minéral argileux à structure en couches.

8. Procédé de blanchiment de dents, comprenant le fait d'appliquer sur la surface de dents jaunies une solution ou une pâte comprenant un agent de blanchiment, conforme à l'une des revendications précédentes, et le fait d'exposer ladite surface à une lumière, ce qui fait blanchir les dents grâce à l'action photocatalytique qui en résulte.

9. Procédé conforme à la revendication 8, dans lequel la lumière à laquelle on expose ladite surface est de la lumière visible.
